# FASCICULE DE BREVET EUROPEEN

(11) **EP 2 491 100 B1**
(45) Date de publication et mention de la délivrance du brevet: **28.08.2013**
(21) Numéro de dépôt: 10785155.2
(22) Date de dépôt: 21.10.2010
(51) Int. Cl.: C11B 3/00, C11B 3/04, C11B 3/06

(54) **PROCEDE D'EXTRACTION DES INSAPONIFIABLES DE MATIERES PREMIERES RENOUVELABLES**
VERFAHREN ZUR EXTRAIEREN VON UNVERSEIFBAREM ANTEIL VON NACHWACHSENDEN ROHSTOFFEN
PROCESS FOR THE EXTRACTION OF UNSAPONIFIABLE MATTER FROM RENEWABLE RAW MATERIAL.

(30) Priorité: 23.10.2009 FR 0957457
(43) Date de publication de la demande: 29.08.2012
(73) Titulaire: Valagro Carbone Renouvelable Poitou-Charentes, 86022 Poitiers Cedex (FR)
(72) Inventeur: PICCIRILLI, Antoine, F-86000 Poitiers (FR)
(74) Mandataire: Cenatiempo, Julie Adeline Anne
(86) Numéro de dépôt international: PCT/FR2010/052247
(87) Numéro de publication internationale: WO 2011/048339

(56) Documents cités:
- FR-A1- 2 762 512
- GB-A- 988 650
- US-A1- 2004 018 258

## Description

La présente invention se rapporte à un procédé d'extraction des insaponifiables d'une matière première lipidique renouvelable, notamment d'un fruit oléifère, d'une graine oléagineuse ou d'une matière première animale, algale, fongique, levurière.

Par définition, on entend par insaponifiables ou composés mineurs d'un corps gras, l'ensemble des composés qui après saponification totale dudit corps gras, c'est-à-dire sous l'action prolongée d'une base alcaline, demeurent insolubles dans l'eau et solubles dans un solvant organique.

Ces insaponifiables peuvent être par exemple des hydrocarbures saturés ou insaturés, des stérols, des alcools aliphatiques et terpéniques, des tocophérols et tocotriénols ou encore des pigments caroténoïdes et xanthophylles.

Actuellement, les procédés classiques d'extraction des insaponif iables d'un corps gras utilisent généralement comme matière première lipidique les huiles végétales et leurs dérivés et co-produits : des huiles végétales brutes, semi-raffinées ou raffinées, des concentrats d'insaponifiables d'huiles végétales obtenus par distillation moléculaire d'huiles végétales brutes, semi-raffinées ou raffinées, et des échappées de désodorisation.

Ces procédés comprennent le plus souvent une étape de saponification de la matière grasse suivie d'une extraction liquide-liquide à l'aide d'un solvant organique.

Lors de la mise en oeuvre de ces procédés d'extraction, les glycérides de la matière grasse sont détruits en savons. Or, ces savons sont des co-produits qui de par leurs propriétés émulsionnantes se retrouvent souvent entraînés avec la glycérine dans les eaux de lavage qu'ils contaminent en DCO (Demande Chimique en Oxygène). Il est donc nécessaire de procéder à leur acidification en acides gras libres, ce qui est contraignant et ne permet qu'une valorisation à très faible valeur ajoutée en savonnerie ou dans la détergence.

En outre, seules les huiles et les échappées de désodorisation (soja, colza, tournesol, maïs, etc et leurs mélanges) peuvent êtres utilisés comme matière première avec ces procédés connus. Or, il existe de nombreuses matières premières de nature différente, comme par exemple les graines ou fruits oléifères, contenant des insaponifiables de haute pureté présentant des propriétés intéressantes.

Il est connu que ces matières premières peuvent être traitées par d'autres procédés mais ceux-ci ne permettent pas l'extraction sélective de fractions insaponifiables. On peut citer à titre d'exemple la demande FR-29119303 qui décrit une méthode de préparation directe d'esters éthyliques d'acides gras par transestérification en présence d'éthanol, de potasse alcoolique comme catalyseur et de flocons de colza après aplatissage des graines, sans toutefois permettre une extraction des insaponifiables.

Il subsiste donc un besoin pour un procédé permettant d'extraire les insaponifiables de corps gras autres que des huiles, dont la mise en oeuvre est économique et permet de récupérer également des coproduits des glycérides à plus haute valeur ajoutée que les acides gras libres.

Pour y répondre, l'invention a pour objet un procédé d'extraction d'une fraction insaponifiable d'une matière première renouvelable choisie parmi les fruits oléifères, les graines oléagineuses, les graines oléoprotéagineuses, les coques de graines, les amandes oléagineuses, les germes, les noyaux et cuticules de fruits, les matières premières animales, algales, fongiques ou levurières riches en lipides, caractérisé en ce qu'il comprend les étapes suivantes :
a) déshydratation et conditionnement de la matière première renouvelable, ne conduisant à aucune extraction de matière grasse,
b) trituration réactive de la matière première lipidique conditionnée en présence d'un alcool léger et d'un catalyseur,
c) évaporation de l'alcool léger,
d) concentration de la phase liquide de façon à obtenir un concentrat comprenant la fraction insaponifiable diluée dans des esters alkyliques d'acides gras,
e) saponification du concentrat d'insaponifiable,
f) extraction de la fraction insaponifiable du mélange saponifié.

L'invention a également pour objet l'utilisation des insaponifiables et des co-produits obtenus pour la préparation de compositions cosmétiques, pharmaceutiques et/ou compléments alimentaires les contenant.

Avantageusement ce procédé permet d'obtenir des insaponifiables à partir de matières premières jamais utilisées jusqu'alors directement, c'est-à-dire sans extraction préalable des lipides, ainsi que des coproduits de glycérides à haute valeur ajoutée comme des esters alkyliques d'acides gras, de la glycérine ainsi que des tourteaux valorisables en alimentation animale. En outre, le procédé selon l'invention est économique car il ne nécessite pas les investissements lourds des procédés classiques. Il est également moins énergivore et nécessite une consommation en eau douce moindre en comparaison des opérations de raffinage des huiles brutes.

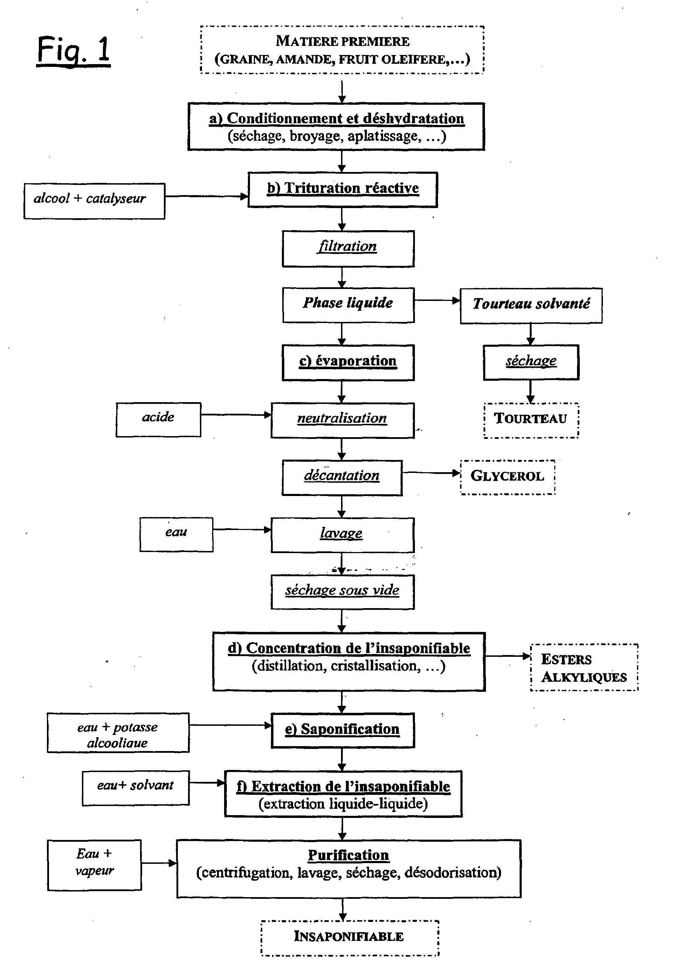

L'invention est maintenant décrite en détail en regard de la figure 1 annexée sur laquelle est représentée un schéma d'un mode de réalisation particulier du procédé d'extraction d'une fraction insaponifiable d'une matière première renouvelable.

L'invention vise donc un procédé d'extraction d'une fraction insaponifiable d'une matière première renouvelable lipidique. Cette matière première est choisie parmi les fruits oléifères, les graines oléagineuses, les graines oléaprotéagineuses, les coques de graines, les amandes oléagineuses, les germes, les noyaux et cuticules de fruits, les matières premières animales, algales, fongiques ou levurières riches en lipides.

Selon un premier mode de réalisation la matière première est un fruit oléifère, en particulier un fruit oléifère choisi parmi l'olive, le karité, la palme ou l'avocat. Si le fruit oléifère est l'avocat, il peut être utilisé frais ou chauffé pour faire apparaître les composés furanniques caractéristiques de l'avocat traité thermiquement.

Selon un deuxième mode de réalisation, la matière première est une graine, une amande, un germe, une cuticule ou un noyau d'une matière première végétale choisie parmi le colza, le soja, le tournesol, le coton, le blé, le maïs, le riz, le raisin, le noyer, le noisetier, le lupin, la cameline, le lin, le carthame, le coprah, l'arachide, le jatropha, le ricin, le neem, le chanvre, le cuphéa, le lesquerella, l'inca inchi, le perilla, l'echium, l'onagre, la bourrache, le cassis, le pin de Corée, le coton, le sésame, l'amaranthe, le café, l'avoine, la tomate, le tagète, le buritti.

La matière première lipidique peut également être une matière première animale, une algue, un champignon, une levure ou une moisissure. Parmi les matières premières animales on préférera le foie et la peau de poisson, tout particulièrement ceux de requin, de morue et de chimère, ainsi que les déchets solides de l'industrie de la viande.

Le procédé selon l'invention comprend une première étape a) de déshydratation et de conditionnement de la matière première renouvelable. La déshydratation peut être mise en oeuvre avant ou après le conditionnement. A titre d'exemple, les fruits oléifères sont préférentiellement déshydratés avant d'être conditionnés, alors qu'inversement les graines oléagineuses sont d'abord aplaties ou broyées avant déshydratation.

Par déshydratation on entend l'élimination totale ou partielle de l'eau de la matière première. Très préférentiellement la matière première lipidique est déshydratée de façon à ce que l'humidité résiduelle soit inférieure ou égale à 3% en poids de matière sèche.

La déshydratation peut être réalisée par exemple par séchage sur lit fluidisé, par séchage sous courant d'air chaud sur lit fixe ou encore par distillation azéotropique, ou tout autre moyen connu de l'homme du métier.

La matière première est conditionnée par la mise en oeuvre d'une étape de conditionnement ne conduisant à aucune extraction de matière grasse.

Préférentiellement la matière première renouvelable est conditionnée par applatissage, floconnage, soufflage ou broyage sous forme de poudre. A titre d'exemple, la matière première peut être toastée ou floconnée, ou encore conditionnée par lyophilisation, per-évaporation, atomisation, broyage mécanique, cryobroyage, flash-détente (séchage rapide par mise sous vide et décompression rapide), aplatissage sur aplatisseur à rouleaux, soufflage par introduction d'air chaud ou de vapeur surchauffée.

Une fois déshydratée et conditionnée, la matière première subie une étape b) de trituration réactive de la matière en présence d'un alcool léger et d'un catalyseur.

Par trituration réactive on entend toute opération visant à transformer les lipides saponifiables en esters alkyliques d'acides gras et en glycérol, préférentiellement en présence d'un ou plusieurs éléments réactifs. Dans le cas présent la trituration est réalisée en présence d'un alcool léger et d'un catalyseur.

La présence de l'alcool léger permet de transformer les glycérides en esters alkyliques.

Préférentiellement l'alcool léger est choisi parmi le méthanol, l'éthanol, le propanol, l'isopropanol, le butanol, le pentanol, l'hexanol, l'éthyl-2-hexanol et leurs isomères.

De même le catalyseur est de façon préférée un catalyseur basique choisi parmi la soude alcoolique, la soude solide, la potasse alcoolique, la potasse solide, le méthylate de sodium ou de potassium, l'éthylate de sodium ou de potassium, le propylate de sodium et de potassium, l'isopropylate de sodium et de potassium, les amines et les polyamines, ou un catalyseur acide choisi parmi l'acide sulfurique, l'acide nitrique, l'acide paratoluènesulfonique, l'acide chlorhydrique et les acides de Lewis.

Cette étape b) peut être réalisée notamment dans un réacteur batch à lit agité ou dans un réacteur continu à tapis mobile du type extracteur continu.

L'étape de trituration réactive permet de récupérer d'une part une phase liquide et d'autre part un tourteau solvanté.

Le tourteau solvanté peut être séché, puis être directement utilisé notamment en alimentation animale.

La phase liquide subit ensuite une étape d'évaporation de l'alcool léger. L'alcool léger doit en effet être évaporé afin de récupérer les lipides.

Après évaporation, le produit lipidique obtenu peut subir une étape de neutralisation, préférentiellement par un acide, puis une étape de décantation qui permet de récupérer du glycérol d'une part et une phase lipidique.

La phase lipidique doit ensuite être concentrée de façon à obtenir d'un côté un concentrat comprenant la fraction insaponifiable diluée dans les esters alkyliques d'acides gras, et d'un autre côté des esters alkyliques de haute pureté, c'est-à-dire des esters limpides et incolores présentant une teneur en esters supérieure à 98% et une teneur en insaponifiables inférieure à 0,1%. Cette fraction d'esters de haute pureté présentant une teneur en esters supérieure à 98% peut être utilisée directement notamment en cosmétique ou en pharmacie.

L'étape de concentration peut en particulier être réalisée par distillation ou par cristallisation. Par distillation on entend toute technique connue de l'homme du métier notamment la distillation moléculaire, la distillation à pression atmosphérique ou sous vide, multi-étagée en série, la distillation azéotropique, l'hydrodistillation, la désodorisation en présence de vapeur ou d'un gaz inerte (azote, gaz carbonique).

La distillation permet d'obtenir une fraction légère appelée distillat comprenant des esters alkyliques de haute pureté et une fraction lourde appelée résidu comprenant la fraction insaponifiable diluée dans les esters alkyliques d'acides gras.

Cette étape peut être réalisée par exemple dans un distillateur moléculaire centrifuge ou à film raclé, dans un évaporateur à film raclé ou à flot tombant, ou encore dans un désodoriseur couche-mince fonctionnant sous vide avec ou sans injection de vapeur, d'azote ou de gaz carbonique.

Le concentrat (la fraction lourde dans le cas d'une distillation) subit ensuite une saponification, c'est-à-dire une réaction chimique transformant un ester en un ion carboxylate hydrosoluble et un alcool.

On extrait ensuite la fraction insaponifiable du mélange saponifié. Préférentiellement cette étape est réalisée par extraction liquide-liquide à l'aide d'un solvant organique.

Le solvant organique peut notamment être un solvant organique de synthèse choisi parmi les alcanes, les aromatiques, les halogéno-alcanes, les éthers et les cétones, ou un solvant organique d'origine naturelle choisi parmi les terpènes tels que le limonène, l'alpha pinène, le bêta pinène, le myrcène, le linalol, le citronellol, le géraniol, le menthol, le citral, le citronellal, ou les dérivés organiques oxygénés notamment les éthers, aldéhydes, alcools et esters tels que par exemple le furfural et le furfurol.

L'extraction peut être réalisée sur une colonne d'extraction à co ou à contre courant ou encore à l'aide d'une batterie de mélangeurs décanteurs.

Une fois extraite, la fraction insaponifiable est préférentiellement purifiée, en particulier par désolvantation, lavage, séchage et/ou désodorisation sous vide. L'étape de purification peut notamment être réalisée par la mise en oeuvre des sous-étapes suivantes :
- centrifugation de la phase solvant de façon à extraire les savons résiduels,
- lavage à l'eau de la phase solvant,
- évaporation sous vide du solvant par distillation sous vide, par hydrodistillation ou par distillation azéotropique,
- désodorisation sous vide de la fraction insaponifiable afin d'en extraire dans les conditions de désodorisation, tout contaminant restant notamment le solvant d'extraction, les pesticides, les hydrocarbures aromatiques polycycliques.

Le procédé selon l'invention permet d'obtenir des insaponifiables purs et d'origine différente de celle classiquement proposée.

De manière non exhaustive, les composés mineurs ou insaponifiables obtenus selon l'invention sont les stérols, les tocophérols, les tocotriénols, le squalène, les polycosanols, les alcools triterpéniques, les polyols gras et composés furanniques de l'avocat, la sésamine et sésamoline, le bêta-carotène, le lycopène, la lutéine, l'asthaxanthine, la co-enzyme Q10, le calciférol, le cholecalciférol, les alkylglycérols, les limonoïdes, l'azadirachtine.

Un exemple particulier de procédé selon l'invention est illustré sur la figure 1.

La matière première est d'abord déshydratée et conditionnée ou conditionnée et déshydratée (étape a).

Elle subit ensuite une trituration réactive en présence d'un alcool léger et d'un catalyseur (étape b). Le produit obtenu est filtré de façon à récupérer d'une part un tourteau solvanté et d'autre part une phase liquide lipidique.

Le tourteau solvanté est séché.

La phase liquide lipidique subit une étape d'évaporation de l'alcool léger (étape c), puis une neutralisation à l'aide d'un acide et une décantation qui permet de récupérer du glycérol. La phase lipidique restante est lavée à l'eau et séchée sous vide.

L'étape suivante est une étape de concentration de l'insaponifiable (étape d) de façon à récupérer des esters alkyliques de haute pureté d'un côté et un concentrat de l'autre.

Le concentrat est ensuite saponifié à l'aide de potasse alcoolique (étape e), puis on réalise une extraction de la fraction insaponifiable (étape f) avec l'eau et un autre solvant.

La fraction insaponifiable est finalement purifiée, par centrifugation, lavage, séchage et désodorisation.

L'invention présente de nombreux avantages par rapport aux procédés classiques existants utilisés pour l'extraction à partir d'huiles ou d'échappées de désodorisation. En terme d'investissement tout d'abord, le procédé selon l'invention permet de s'affranchir des outils de trituration mécanique comme une presse à vis ou un extracteur à l'hexane, et des outils de raffinage (démucilagination, neutralisation). En outre, contrairement à la trituration mécanique ou évaporative à l'hexane, et au raffinage, la trituration réactive selon l'invention n'entraîne pas de fortes consommations d'énergie. Elle n'entraîne pas non plus de consommation d'eau douce à l'inverse des opérations de raffinage des huiles brutes.

De plus, l'invention est très avantageuse en terme de co-valorisation car la mise en oeuvre du procédé conduit à des co-produits tels que :
- des esters alkyliques de pureté élevée, directement valorisables en cosmétique ou en pharmacie,
- de la glycérine qui trouve des applications en cosmétique, pharmacie, hygiène, fluides anti-gels, etc., et
- des tourteaux directement valorisables en alimentation animale.

La présente invention a également pour objet l'utilisation d'une fraction insaponifiable ou de co-produits obtenus par la mise en oeuvre du procédé pour la préparation d'une composition cosmétique, médicamenteuse, alimentaire ou compléments ou additifs alimentaires. Avantageusement cette fraction insaponifiable et les co-produits obtenus selon l'invention ne contiennent pas de résidus de solvant toxique et présentent une bien meilleure innocuité et acceptabilité réglementaire (liée à l'absence de solvant résiduel toxique), que les produits obtenus par la mise en oeuvre de procédés classiques. Ces caractéristiques particulières permettent une utilisation plus adaptée comme composition cosmétique, médicamenteuse, alimentaire ou compléments ou additifs alimentaires.

L'invention est maintenant illustrée au travers de différents exemples.

### EXEMPLE 1 : EXTRACTION DES COMPOSES MINEURS DE LA GRAINE DE SESAME

Pour ce premier essai, la matière première utilisée est une graine de sésame présentant les caractéristiques suivantes :
- Teneur en eau : 8 % en poids de matière sèche,
- Teneur en matière grasse : 45,0 % en poids de matière sèche.

Après extraction à l'aide d'un appareillage de type soxhlet selon la méthode NF ISO 659, l'analyse de l'huile extraite selon la méthode NFT 60-205-1, révèle une teneur en fraction insaponifiable de 0,8% en poids de matière grasse. L'analyse en chromatographie couche mince de l'insaponifiable réalisée en présence d'un mélange éluant hexane/éther (2/1 volumique) révèle la présence comme composés majoritaires de sésamine, de sésamoline et de phytostérols. De même, l'acidité de l'huile de la graine déterminée par la méthode NFT 60-204, est de 5,0 mg KOH/g.

Dans une première étape, 1000 g de graines non décortiquées sont séchés à l'étude à 80°C pendant 24 heures. L'humidité finale de la graine est de 0,8% en poids de matière sèche. Les graines séchées sont alors aplaties à l'aide d'un aplatisseur à cylindres afin d'obtenir 878 g de flocons.

Dans un réacteur en verre de 2 litres équipé d'un réfrigérant à boule, d'une double enveloppe et d'une agitation mécanique, 678 g de flocons sont alors mis en présence de 1756 g d'éthanol anhydre et de 8,4 g de soude en écailles préalablement dissous dans 200 g d'éthanol anhydre. Le mélange est porté sous agitation à 55°C pendant 30 minutes. Le mélange est ensuite filtré sur Büchner et le gâteau lavé avec 3 fois 100 ml d'éthanol.

Le filtrat est ensuite neutralisé à 50°C, par ajout de 100 ml d'une solution alcoolique d'acide sulfurique contenant 3 g de H₂SO₄. L'éthanol est alors distillé sous vide (300 mbar) à l'évaporateur rotatif, à une température de 30°C pendant 30 minutes.

Le mélange obtenu est alors diphasique puisque on observe la décantation de la phase glycérinée. La glycérine est alors extraite par centrifugation à 3500 tr/minute. La phase ester obtenue est enfin lavée par ajout d'eau déminéralisée (6 lavages successifs soit pour chaque lavage, 6% d'eau en poids de la phase ester), puis séchée sous vide à l'évaporateur rotatif, à 90°C (20 mbar) pendant 30 minutes. On obtient alors 320 g d'esters éthyliques.

La phase ester est alors envoyée dans un distillateur moléculaire à film raclé de type KDL4. La température est fixée à 140°C et le vide à 0,008 mbar. Le rendement en distillat est 96,2%. La phase lourde chargée en insaponifiable et en esters éthyliques présente une teneur en insaponif iable de 20,5% en poids de matière grasse.

Dans un ballon de 100 ml équipé d'un réfrigérant, on place la phase lourde (11,5 g) en présence de 48 ml de potasse alcoolique 3,7M et de quelques billes de verre. Le mélange est alors porté à reflux (95°C) pendant 4 heures. Après refroidissement, le milieu est dilué avec 75 ml d'eau déminéralisée. Le mélange hydro-alcoolique obtenu est alors extrait par le limonène (solvant d'extraction) fourni par la société Interchimie.

L'extraction est réalisée à l'ampoule à décanter (500 ml). Le milieu saponifié est introduit dans l'ampoule à décanter avec 100 ml de limonène issu de l'opération de rinçage du ballon de saponification. Après décantation, la phase organique est séparée de la phase aqueuse, cette dernière étant reprise à nouveau par 100 ml de limonène. La phase aqueuse subit ainsi 4 extractions successives en présence à chaque fois de 100 ml de limonène.

Les phases organiques sont alors rassemblées puis lavées par ajout d'eau déminéralisée jusqu'à neutralité (contrôle au papier pH). Chaque lavage est réalisé par ajout de 100 ml d'eau.

Le solvant limonène est alors éliminé par distillation sous vide : à 140°C sous 20 mbar. Le résidu de distillation obtenu, constitué par les composés insaponif iables initialement présents dans la phase lourde obtenue par distillation moléculaire est alors pesé précisément puis analysé par chromatographie couche mince (CCM). On obtient 2,1 g de composés insaponifiables.

Le rendement global du procédé d'extraction des insaponifiables est de 63%.

Le tableau ci-dessous rassemble les résultats d'analyse par chromatographie en couche mince (CCM) des fractions insaponifiables de l'huile de sésame présente dans la graine (obtenu par la méthode NFT 60-205-1) et de l'insaponifiable extrait au cours du procédé de trituration réactive.

| **Analyse et observations** | **Fractions insaponifiables de sésame** | |
|---|---|---|
| | **Méthode NFT 60-205-1** | **Procédé de trituration réactive des graines** |
| *Analyse CCM* | | |
| Intensité des spots de stérols | ++++ | ++++ |
| Intensité des spots de sésamine et de sésamoline | ++++ | ++++ |
| Intensité des spots de tocophérols | ++ | ++ |

Les résultats du tableau 1 indiquent que le procédé de trituration réactive des graines permet d'obtenir avec un rendement appréciable, une fraction insaponifiable présentant une composition très proche, voire identique à celle de l'insaponif iable présent dans les lipides de la graine (méthode NFT 60-205-1). De même, les esters éthyliques obtenus dans le distillat de distillation moléculaire sont limpides et d'aspect incolore et présentent une acidité finale de 0,5 mg KOH/g ainsi qu'une pureté en esters éthyliques de 99,3%. De par leur qualité et leur aspect, ces esters constituent un coproduit valorisable du procédé pouvant être destiné à des applications cosmétiques et pharmaceutiques.

### EXEMPLE 2 : EXTRACTION DES COMPOSES MINEURS DE L'AVOCAT

Pour ce premier essai, la matière première utilisée est 10 kg d'avocat frais de la variété Haas présentant les caractéristiques suivantes :
- Teneur en eau : 91,7 %
- Teneur en matière grasse de la matière sèche : 38,3 % en poids de matière sèche
- Acidité de la matière grasse : 2,1 mg KOH/g, et
- Teneur en insaponifiable de la matière grasse : 4,2 % en poids de matière grasse.

Dans une première étape, les avocats frais sont coupés en rondelles (noyau compris). Les rondelles sont ensuite séchées sous air à l'étuve ventilée à 70°C pendant 36 heures. L'humidité résiduelle de l'avocat tranché est de 6,3 % en poids de matière grasse. Les tranches sont alors broyées au mixeur en vue d'obtenir une poudre de granulométrie comprise entre 0,2 et 0,4 mm. Cette poudre est alors lyophilisée jusqu'à atteindre une humidité inférieure à 3 % en poids de matière grasse. On obtient ainsi 758 g de poudre d'avocat sec (humidité résiduelle de 0,9 % en poids de matière grasse).

Dans une seconde étape, ces 758 g d'avocat en poudre sont placés dans un réacteur en verre de 5 litres, équipé d'un réfrigérant à boule, d'une double enveloppe et d'une agitation mécanique, en présence de 2000 g d'éthanol anhydre et de 9,9 g de soude en écailles préalablement dissoutes dans 240 g d'éthanol anhydre. Le mélange est porté sous agitation à 55°C pendant 45 minutes. Le mélange est ensuite filtré sur Büchner et le gâteau lavé avec 5 fois 120 ml d'éthanol.

Le filtrat est ensuite neutralisé à 50°C, par ajout de 100 ml d'une solution alcoolique d'acide sulfurique contenant 3,5 g de H₂SO₄. L'éthanol est alors distillé sous vide (500 mbar) à l'évaporateur rotatif et à une température de 70°C.

Le mélange obtenu est alors diphasique puisque on observe la décantation de la phase glycérinée. La glycérine est alors extraite par centrifugation à 3500 tr/minute. La phase ester obtenue est enfin lavée par ajout d'eau déminéralisée (6 lavages successifs soit pour chaque lavage, 6% d'eau en poids de la phase ester), puis séchée sous vide à l'évaporateur rotatif, à 90°C (20 mbar) pendant 90 minutes. On obtient alors 259 g de phase grasse (esters éthyliques). On procède alors à une détermination de la teneur en insaponifiable de la phase ester, selon la méthode NFT 60-205-1 dans laquelle l'hexane a été remplacé par le 1,2 dichloroéthane. Aussi, une analyse par CCM de l'insaponifiable est réalisée, mettant en évidence les composés caractéristiques de l'insaponifiable d'avocat de fruits modérément séchés : composés céto-hydroxylés, lipides furanniques, polyols gras, stérols.

La phase ester est alors envoyée dans un distillateur moléculaire à film raclé de type KDL4. La température est fixée à 140°C et le vide à 0,008 mbar. Le rendement en distillat est de 88,3%. La phase lourde, chargée en insaponifiable et en esters éthyliques, présente une teneur en insaponifiable de 35,2 %/MG. Dans un ballon de 100 ml équipé d'un réfrigérant, on place la phase lourde en présence de 150 ml de potasse alcoolique 3,7M et de quelques billes de verre. Le mélange est alors porté à reflux (95°C) pendant 4 heures. Après refroidissement, le milieu est dilué avec 150 ml d'eau déminéralisée. Le mélange hydro-alcoolique obtenu est alors extrait par le limonène (solvant d'extraction) fourni par la société Interchimie.

L'extraction est réalisée en plusieurs fois à l'ampoule à décanter. Le milieu saponifié est introduit dans l'ampoule à décanter avec 100 ml de limonène issu de l'opération de rinçage du ballon de saponification. Après décantation, la phase organique est séparée de la phase aqueuse, cette dernière étant reprise à nouveau par 100 ml de limonène. La phase aqueuse subit ainsi 4 extractions successives en présence à chaque fois de 100 ml de solvant.

Les phases organiques sont alors rassemblées puis lavées par ajout d'eau déminéralisée jusqu'à neutralité (contrôle au papier pH). Chaque lavage est réalisé par ajout de 100 ml d'eau.

Le solvant limonène est alors éliminé par distillation sous vide : à 140°C sous 20 mbar. Le résidu de distillation obtenu, constitué par les composés insaponifiables initialement présents dans la phase lourde obtenue par distillation moléculaire est alors pesé précisément puis analysé par chromatographie en couche mince (CCM). On obtient 8,7 g de composés insaponif iables.

Le rendement global du procédé d'extraction des insaponifiables est de 72%.

Le tableau suivant rassemble les résultats d'analyse CCM des fractions insaponifiables de la phase grasse avant extraction (analyse des esters éthyliques selon la méthode NFT 60-205-1 modifiée en remplaçant l'hexane par le 1,2 dichloroéthane) et de l'insaponifiable extrait au cours du procédé de trituration réactive.

| **Analyse et observations** | **Fractions insaponifiables de l'avocat** | |
|---|---|---|
| | **Méthode NFT 60-205-1 (1)** | **Procédé de trituration réactive de l'avocat deshydraté** |
| *Analyse CCM* | | |
| Intensité des spots de stérols | + | + |
| Intensité des spots de lipides furanniques | ++ | ++ |
| Intensité des spots des composés céto-hydroxylés | Traces | Non Traces |
| Composés non identifiés | ++ | ++ |
| Intensité des spots d'alcools gras polyhydroxylés | +++ | +++ |

### (1) méthode modifiée en remplaçant l'hexane par le 1,2 dichloroéthane

Les résultats du tableau ci-dessus indiquent que le procédé de trituration réactive mis en oeuvre sur l'avocat séché permet d'obtenir avec un rendement appréciable (72%), une fraction insaponif iable présentant une composition très proche, voire identique à celle de l'insaponifiable d'avocat obtenu à partir de fruits séchés.

De même, les esters éthyliques d'avocat obtenus dans le distillat de distillation moléculaire sont d'aspect incolore et présentent une acidité finale de 0,3 mg KOH/g ainsi qu'une teneur en esters éthyliques de 99,6%. De par leur qualité et leur aspect, ces esters constituent un coproduit valorisable du procédé pouvant être destiné à des applications cosmétiques et pharmaceutiques.

## Revendications

1. Procédé d'extraction d'une fraction insaponifiables d'une matière première renouvelable choisie parmi les fruits oléifères, les graines oléagineuses, les graines oléoprotéagineuses, les coques de graines, les amandes oléagineuses, les germes, les noyaux et cuticules de fruits, les matières premières animales, algales, fongiques ou levurières riches en lipides, **caractérisé en ce qu'**il comprend les étapes suivantes :
a) déshydratation et conditionnement de la matière première renouvelable, ne conduisant à aucune extraction de matière grasse,
b) trituration réactive de la matière première lipidique conditionnée en présence d'un alcool léger et d'un catalyseur, de façon à récupérer d'une part une phase liquide et d'autre part un tourteau solvanté,
c) évaporation de l'alcool léger de la phase liquide,
d) concentration de la phase liquide de façon à obtenir un concentrat comprenant la fraction insaponifiable diluée dans des esters alkyliques d'acides gras,
e) saponification du concentrat d'insaponifiable,
f) extraction de la fraction insaponifiable du mélange saponifié.

2. Procédé d'extraction d'une fraction insaponif iable selon la revendication 1, **caractérisé en ce que** la matière première renouvelable est un fruit oléifère choisi parmi l'olive, le karité, la palme, l'avocat frais ou chauffé.

3. Procédé d'extraction d'une fraction insaponifiable selon la revendication 1, **caractérisé en ce que** la matière première renouvelable est une graine, une amande, un germe, une cuticule ou un noyau d'une matière première végétale choisie parmi le colza, le soja, le tournesol, le coton, le blé, le maïs, le riz, le raisin, le noyer, le noisetier, le lupin, la cameline, le lin, le carthame, le coprah, l'arachide, le jatropha, le ricin, le neem, le chanvre, le cuphéa, la lesquerella, l'inca inchi, la perilln, l'echium, l'onagre, la bourrache, le cassis, le pin de Corée, le coton, le sésame, l'amaranthe, le café, l'avoine, la tomate, le tagète, le buritti.

4. Procédé d'extraction d'une fraction insaponifiable selon la revendication 1, **caractérisé en ce que** la matière première renouvelable est du foie ou de la peau de poisson ou un déchet solide de l'industrie de la viande.

5. Procédé d'extraction d'une fraction insaponifiable selon l'une des précédentes revendications, **caractérisé en ce que** la déshydratation est réalisée de façon à atteindre une humidité résiduelle inférieure ou égale à 3% en poids de la matière sèche.

6. Procédé d'extraction d'une fraction insaponifiable selon l'une des précédentes revendications, **caractérisé en ce que** le conditionnement de la matière première est réalisé par aplatissage, floconnage, soufflage, flash détente et/ou broyage.

7. Procédé d'extraction d'une fraction insaponifiable selon l'une des précédentes revendications, **caractérisé en ce que** l'alcool léger est choisi parmi le méthanol, l'éthanol, le propanol, l'isopropanol, le butanol, le pentanol, l'hexanol, l'éthyl-2-hexanol et leurs isomères.

8. Procédé d'extraction d'une fraction insaponifiable selon l'une des précédentes revendications, **caractérisé en ce que** le catalyseur est un catalyseur basique choisi parmi la soude, la soude alcoolique, la soude solide, la potasse, la potasse alcoolique, la potasse solide, le méthylate de sodium ou de potassium, l'éthylate de sodium ou de potassium, le propylate de sodium et de potassium, l'isopropylate de sodium et de potassium, les amines et les polyamines, ou un catalyseur acide choisi parmi l'acide sulfurique, l'acide nitrique, l'acide paratoluènesulfonique, l'acide chlorhydrique et les acides de Lewis.

9. Procédé d'extraction d'une fraction insaponifiable selon l'une des précédentes revendications, **caractérisé en ce que** l'étape de concentration est réalisée par distillation ou par cristallisation.

10. Procédé d'extraction d'une fraction insaponifiable selon l'une des précédentes revendications, **caractérisé en ce que** l'étape d'extraction est réalisée par extraction liquide-liquide à l'aide d'un solvant organique.

11. Procédé d'extraction d'une fraction insaponifiable selon la revendication 10, **caractérisée en ce que** le solvant organique est un solvant organique de synthèse choisi parmi les alcanes, les aromatiques, les halogénoalcanes, les éthers et les cétones, ou un solvant organique d'origine naturelle choisi parmi les terpènes et les dérivés organiques oxygénés.

12. Procédé d'extraction d'une fraction insaponifiable selon l'une des précédentes revendications, **caractérisé en ce qu'**il comporte une étape supplémentaire de purification consistant en la succession des sous-étapes suivantes :
- centrifugation de la phase solvant de façon à extraire les savons résiduels,
- lavage à l'eau de la phase solvant contre-courant,
- évaporation sous vide du solvant par distillation sous vide, par hydrodistillation ou par distillation azéotropique,
- désodorisation sous vide de la fraction insaponifiable afin d'en extraire tout contaminant volatil restant.

## Patentansprüche

1. Verfahren zum Extrahieren eines unverseifbaren Anteils eines nachwachsenden Rohstoffs, der unter ölhaltigen Früchten, ölhaltigen Samenkörnern, öl- und proteinhaltigen Samenkörnern, Schalen von Samenkörnern, ölhaltigen Mandeln, Keimen, Kernen, Kutikulas von Früchten oder aus an Lipiden reichen Rohstoffen aus Tieren, Algen, Pilzen, Hefen ausgewählt wird, **dadurch gekennzeichnet, dass** das Verfahren folgende Verfahrensschritte umfasst:
a) Dehydrierung und Aufbereitung des nachwachsenden Rohstoffs ohne Extraktion von fetthaltigem Material,
b) reaktives Zerreiben des aufbereiteten fetthaltigen Rohstoffs in Anwesenheit eines leichten Alkohols und eines Katalysators um einerseits eine flüssige Phase und andererseits gelöste ölrückstande zu gewinnen,
c) Verdampfung des leichten Alkohols aus der flüssigen Phase,
d) Konzentration der flüssigen Phase, um ein Konzentrat zu erhalten, das den unverseifbaren Anteil enthält, der in Alkylestern von Fettsäuren gelöst ist,
e) Verseifung des unverseifbaren Konzentrats,
f) Extraktion des unverseifbaren Anteils der verseiften Mischung.

2. Verfahren zum Extrahieren eines unverseifbaren Anteils nach Anspruch 1, **dadurch gekennzeichnet, dass** der nachwachsende Rohstoff eine ölhaltige Frucht ist, die unter Olive, Karitébaumfrucht, Palmfrucht, frischer oder erhitzter Avocado ausgewählt ist.

3. Verfahren zum Extrahieren eines unverseifbaren Anteils nach Anspruch 1, **dadurch gekennzeichnet, dass** der nachwachsende Rohstoff ein Samenkorn, eine Mandel, ein Keim, eine Kutikula oder ein Kern eines pflanzlichen Rohstoffs ist, der unter Raps, Soja, Sonnenblume, Baumwolle, Weizen, Mais, Reis, Weintrauben, Walnussbaum, Haselnussstrauch, Lupine, Dotterblume, Flachs, Färberdistel, Kopra, Erdnuss, Jatropha, Rizinus, Niembaum, Hanf, Cuphea, Lesquerella, Inca Inchi, Perilla, Echium, Nachtkerze, Gurkenkraut, Johannisbeere, Koreakiefer, Baumwolle, Sesam, Amarant, Kaffee, Hafer, Tomate, Tagetes und Buriti-Palme ausgewählt ist.

4. Verfahren zum Extrahieren eines unverseifbaren Anteils nach Anspruch 1, **dadurch gekennzeichnet, dass** der nachwachsende Rohstoff eine Leber oder eine Haut eines Fisches oder ein fester Abfall der Fleischindustrie ist.

5. Verfahren zum Extrahieren eines unverseifbaren Anteils nach einem der vorhergehenden Ansprüche, **dadurch gekennzeichnet, dass** die Dehydrierung derart durchgeführt wird, dass eine Restfeuchte unterhalb oder gleich 3 Gew.-% der Trockenmasse erreicht wird.

6. Verfahren zum Extrahieren eines unverseifbaren Anteils nach einem der vorhergehenden Ansprüche, **dadurch gekennzeichnet, dass** die Aufbereitung des Rohstoffs durch Flachdrücken, Flockenbildung, Blasen, Vakuumextraktion und/oder Zerkleinerung bewerkstelligt wird.

7. Verfahren zum Extrahieren eines unverseifbaren Anteils nach einem der vorhergehenden Ansprüche, **dadurch gekennzeichnet, dass** der leichte Alkohol unter Methanol, Ethanol, Propanol, Isopropanol, Butanol, Pentanol, Hexanol, Ethyl-2-hexanol und deren Isomeren ausgewählt wird.

8. Verfahren zum Extrahieren eines unverseifbaren Anteils nach einem der vorhergehenden Ansprüche, **dadurch gekennzeichnet, dass** der Katalysator ein basischer Katalysator ist, der unter Soda, alkoholischem Soda, festem Soda, Pottasche, alkoholischer Pottasche, fester Pottasche, Methylat von Natrium oder von Kalium, Ethylat von Natrium oder Kalium, Propylat von Natrium oder von Kalium, Isopropylat von Natrium oder Kalium, den Aminen oder den Polyaminen ausgewählt ist, oder ein saurer Katalysator ist, der unter Schwefelsäure, Salpetersäure, p-Toluolsulfonsäure, Salzsäure oder den LewisSäuren ausgewählt ist.

9. Verfahren zum Extrahieren eines unverseifbaren Anteils nach einem der vorhergehenden Ansprüche, **dadurch gekennzeichnet, dass** der Verfahrensschritt der Konzentration durch Destillation oder Kristallisation ausgeführt wird.

10. Verfahren zum Extrahieren eines unverseifbaren Anteils nach einem der vorhergehenden Ansprüche, **dadurch gekennzeichnet, dass** der Verfahrensschritt der Extraktion durch eine Flüssig-Flüssig-Extraktion mithilfe eines organischen Lösungsmittels durchgeführt wird.

11. Verfahren zum Extrahieren eines unverseifbaren Anteils nach Anspruch 10, **dadurch gekennzeichnet, dass** das organische Lösungsmittel ein synthetisches organisches Lösungsmittel ist, das unter den Alkanen, den Aromaten, den Halogenalkanen, den Estern und den Ketonen ausgewählt ist, oder ein organisches Lösungsmittel natürlichen Ursprungs ist, das unter den Terpenen und den oxidierten organischen Derivaten ausgewählt ist.

12. Verfahren zum Extrahieren eines unverseifbaren Anteils nach einem der vorhergehenden Ansprüche, **dadurch gekennzeichnet, dass** das Verfahren zusätzlich einen Verfahrensschritt der Reinigung aufweist, der die Folge der Unterverfahrensschritte aufweist:
- Zentrifugieren der Lösungsphase um die verbleibenden Seifen zu extrahieren,
- Gegenstromwaschen der Lösungsphase, mit Nasser
- Verdampfung unter Vakuum des Lösungsmittels durch Destillation unter Vakuum, durch Hydrodestillation oder durch azeotrope Destillation,
- Desodorierung unter Vakuum des unverseifbaren Anteils, um daraus volatile kontaminierende Reste zu entfernen.

## Claims

1. Process for extraction of an unsaponifiable fraction of a renewable raw material that is selected from among oil-producing fruits, oleaginous seeds, oleoproteaginous seeds, seed husks, oleaginous almonds, sprouts, pits and cuticles of fruits, and lipid-rich animal, algal, fungal or yeast raw materials, **characterized in that** it comprises the following stages:
a) Dehydration and packaging of the renewable raw material, not leading to any extraction of fat,
b) Reactive trituration of the packaged lipidic raw material in the presence of a light alcohol and a catalyst, in such a way as to recover, on the one hand, a liquid phase, and, on the other hand, a solvent-containing meal,
c) Evaporation of light alcohol from the liquid phase,
d) Concentration of the liquid phase so as to obtain a concentrate that comprises the dilute unsaponifiable fraction in fatty acid alkyl esters,
e) Saponification of unsaponifiable concentrate,
f) Extraction of the unsaponifiable fraction from the saponified mixture.

2. Process for extraction of an unsaponifiable fraction according to Claim 1, wherein the renewable raw material is an oil-producing fruit that is selected from among olive, shea, palm or avocado that is fresh or heated up.

3. Process for extraction of an unsaponifiable fraction according to Claim 1, wherein the renewable raw material is a seed, an almond, a sprout, a cuticle or a pit of a plant raw material that is selected from among canola, soy, sunflower, cotton, wheat, corn, rice, grape, walnut, hazelnut, lupine, camelina, flax, safflower, copra, peanut, jatropha, castor oil, neem, hemp, cuphea, lesquerella, inca inchi, perilla, echium, evening primrose, borage, blackcurrant, pine of Korea, cotton, sesame, amaranth, coffee, oats, tomato, marigold, and buriti.

4. Process for extraction of an unsaponifiable fraction according to Claim 1, wherein the renewable raw material is liver or fish-skin, or solid waste from the meat industry.

5. Process for extraction of an unsaponifiable fraction according to one of the preceding claims, wherein the dehydration is carried out in such a way as to reach a residual moisture that is less than or equal to 3% by weight of the dry material.

6. Process for extraction of an unsaponifiable fraction according to one of the preceding claims, wherein the packaging of the raw material is done by flattening, flaking, blowing, flash-expansion and/or grinding.

7. Process for extraction of an unsaponifiable fraction according to one of the preceding claims, wherein the light alcohol is selected from among methanol, ethanol, propanol, isopropanol, butanol, pentanol, hexanol, ethyl-2-hexanol and isomers thereof.

8. Process for extraction of an unsaponifiable fraction according to one of the preceding claims, wherein the catalyst is a basic catalyst that is selected from among soda, alcoholic soda, solid soda, potash, alcoholic potash, solid potash, sodium or potassium methylate, sodium or potassium ethylate, sodium and potassium propylate, sodium and potassium isopropylate, alnines and polyamines, or an acid catalyst that is selected from among sulfuric acid, nitric acid, paratoluenesulfonic acid, hydrochloric acid and Lewis acids.

9. Process for extraction of an unsaponifiable fraction according to one of the preceding claims, wherein the concentration stage is carried out by distillation or by crystallization.

10. Process for extraction of an unsaponifiable fraction according to one of the preceding claims, wherein the extraction stage is carried out by liquid-liquid extraction using an organic solvent.

11. Process for extraction of an unsaponifiable fraction according to Claim 10, wherein the organic solvent is an organic synthesis solvent that is selected from among alkanes, aromatic compounds, halo-alkanes, ethers and ketones, or an organic solvent of natural origin that is selected from among terpenes and oxidized organic derivatives.

12. Process for extraction of an unsaponifiable fraction according to one of the preceding claims, wherein it comprises an additional purification stage that consists of the series of the following sub-stages:
- Centrifuging of the solvent phase in such a way as to extract residual soaps,
- Washing the counter-current solvent phase with water,
- Vacuum evaporation of the solvent by vacuum distillation, by hydrodistillation, or by azeotropic distillation,
- Vacuum deodorization of the unsaponifiable fraction so as to extract from it any remaining volatile contaminant.
